# EUROPEAN PATENT SPECIFICATION

(11) **EP 1 623 715 B1**
(45) Date of publication and mention of the grant of the patent: **19.09.2012**
(21) Application number: 04016497.2
(22) Date of filing: 13.07.2004
(51) Int. Cl.: A61K 47/48, A61K 36/36, A61P 35/00

(54) **A composition comprising a pharmacologically active agent coupled to a target cell specific component, and a saponin**
Zusammensetzung, welche ein pharmakologisch aktives, an eine für eine Zielzelle spezifische Komponente gebundenes Mittel sowie ein Saponin enthält
Composition comportant un agent pharmacologiquement actif couplé à un élément de ciblage spécifique de cellules et une saponine

(43) Date of publication of application: 08.02.2006
(73) Proprietor: Charité - Universitätsmedizin Berlin, 10117 Berlin (DE); Freie Universität Berlin, 14195 Berlin (DE)
(72) Inventor: Fuchs, Hendrik, 13353 Berlin (DE); Sutherland, Mark, 12200 Berlin (DE); Bachran, Christopher, 13349 Berlin (DE); Melzig, Matthias, 12623 Berlin (DE); Heisler, Iring, 14199 Berlin (DE); Hebestreit, Philip, 67157 Wachenheim an der Weinstrasse (DE)
(74) Representative: Engelhard, Markus

(56) References cited:
- WO-A-01/95935
- WO-A-02/072631
- US-A1- 2005 008 717
- HEBESTREIT, P. ET AL: "Cytotoxic activity of the seeds from Agrostemma githago var. githago" PLANTA MED., vol. 69, 2003, pages 921-925, XP008042795
- KELLER J ET AL: "Development of a novel molecular adapter for the optimization of immunotoxins" JOURNAL OF CONTROLLED RELEASE, ELSEVIER SCIENCE PUBLISHERS B.V. AMSTERDAM, NL, vol. 74, no. 1-3, 6 July 2001 (2001-07-06), pages 259-261, XP004297533 ISSN: 0168-3659
- YESILADA E ET AL: "Effects of triterpene saponins from Astragalus species on in vitro cytokine release" JOURNAL OF ETHNOPHARMACOLOGY, ELSEVIER SCIENTIFIC PUBLISHERS LTD, IE, vol. 96, no. 1-2, 4 January 2005 (2005-01-04), pages 71-77, XP004657423 ISSN: 0378-8741
- HEISLER I. ET AL: "A cleavable adapter to reduce nonspecific cytotoxicity of recombinant immunotoxins", INTERNATIONAL JOURNAL OF CANCER, vol. 103, no. 2, 1 January 2003 (2003-01-01), pages 277-282, XP008042788, JOHN WILEY & SONS, INC, UNITED STATES, SWITZERLAND, GERMANY
- BACHRAN C. ET AL: "The saponin-mediated enhanced uptake of targeted saporin-based drugs is strongly dependent on the saponin structure", EXPERIMENTAL BIOLOGY AND MEDICINE, vol. 231, no. 4, April 2006 (2006-04), pages 412-420,

## Description

The present invention relates to a composition comprising a pharmacologically active agent coupled to a target cell specific component, and a saponin according to claim 1, to uses of said composition and to a kit comprising said composition.

The success of a medical treatment using a pharmacologically active agent, in many instances, depends on the ability to deliver such a pharmacologically active agent to its intended site of action. However, quite frequently, one is not able to achieve sufficiently large concentrations of the pharmacologically active agent at the intended site of action, either because the pharmacologically active agent never reaches the site in the first place or is metabolized too quickly without being able to exert its action. One way of approaching this problem in the past has been to combine the pharmacologically agent with a target specific component so as to increase the likelihood of the agent reaching the target. This approach has been for example adopted in the case of immunotoxins. In the broadest sense immunotoxins (IT) are chimeric proteins or chemically coupled conjugates in which a cell-targeting moiety is combined with a cytotoxic agent. The latter is either a small radioactive molecule, a low molecular weight organic compound or the catalytic domain of a natural toxin. Usually the cell targeting moiety is an antibody fragment or a natural ligand for cancer-associated endocytic surface antigens. Typical target antigens are the epidermal growth factor (EGF) receptor, the proto-oncogene receptor ErbB2 (also known as HER-2 in humans), the interleukin-2 receptor or cancer-associated carbohydrates (1).

The first recombinant IT approved by the American Food and Drug Administration was Ontak. It consists of interleukin-2 and parts of diphtheria toxin. It was developed for the treatment of cutaneous T-cell lymphoma (2, 3). In addition to Ontak two further chemically coupled ITs, Zevalin (4, 5) and Mylotarg (6), have been approved and several ITs are under investigation in clinical trials.

In spite of the clinical success of several newly developed ITs the effective cytosolic uptake of the drug remains a main problem requiring the application of large total doses to achieve the high local concentrations needed. This often leads to non-specific toxicity, which often results in diffuse endothelial damage and concomitant vascular leak syndrome (reviewed in (7)). An improvement for recombinant ITs was the development of a molecular adapter linking the toxic and targeting moiety. Due to cleavable endosomal and cytosolic peptides the adapter mediates cytosolic trapping of the toxin (8) which leads to reduced side effects on non-target cells *in vitro* (9).

In some immunotoxins the natural membrane translocating sequence of the toxin is used to mediate endosomal membrane transfer. While this is possible e.g. for diphtheria toxin and *Pseudomonas* exotoxin other toxins like plant type I ribosome-inactivating proteins (RIP) lacking a translocating sequence must enter the cells using other to date unknown mechanisms. In the last decade, the use of protein transduction domains (PTD, also referred to as Trojan peptides or cell permeable proteins) capable of transporting effector molecules into cells has become an increasingly attractive mechanism to solve this problem (reviewed in (10, 11)). Various peptide vectors derived from viral, bacterial, insect, and mammalian proteins possessing membrane transduction properties have been identified and tested for their ability to deliver compounds, proteins and DNA. In all these experiments, however, the PTD-mediated uptake was unspecific and thus independent of the cell type and the cellular surface receptors. To solve this problem a PTD was linked in so called immunoadaptertoxins via an endosomal cleavable peptide to a target cell-specific moiety. The PTD is not exposed until binding of the immunoadaptertoxin to a target cell-specific receptor, internalization and endosomal cleavage (9). Nevertheless, the cytosolic uptake of these improved ITs is clearly lower than that of potent natural toxins.

Insufficient uptake of the drug due to inadequate membrane transfer from the lumen of intracellular compartments to the cytosol is a general problem of ITs. After binding to an endocytic receptor and subsequent internalization most of the ITs are either directed into lysosomes for degradation or recycled back to the cell surface (12). The cytosolic uptake of small organic compounds like 5-fluorouridine can be enhanced by coupling the drug via an acid-cleavable hydrazone bond to a target cell-specific antibody or ligand (13). After internalization the drug is released inside the endosomes by acidic hydrolysis and passively diffuses into the cytosol. This technique, however, allows the drug, once released, to enter other cells non-specifically and is not applicable to large drugs like protein toxins. ITs such as BL22 or Ontak rely on the translocation domain of the natural toxin for effective uptake (14). This limits the number of natural toxins available for the construction of ITs. In order to improve the uptake of toxins that do not possess a translocation domain like diphtheria toxin A-chain, ricin toxin A-chain, the type 1 RIP saporin-3 or toxic human proteins the introduction of membrane permeable peptides, called protein transduction domains (PTD) or Trojan peptides, was suggested (8). Although the PTDs were shown to be active in numerous studies dealing with unspecific protein import, the attempt to enhance the cytotoxicity of immunotoxins were unsuccessful (9, 15). The insertion of two cleavable peptides flanking the PTD, however, has been shown to reduce side effects on non-target cells (9). The efforts to increase the specificity for target cells is usually accompanied by a concomitant loss in the cytotoxic potential in comparison to the native toxin. The effectiveness of natural diphtheria toxin, dependent on the target cell line with an IC₅₀ of 10 to 100 pM (8), was not reached to date.

Hebestreit and Melzig (29) showed that the saponin agrostenumasaponin 1 enhances the cytotoxicity of the ribosome-inactivating protein agrostin despite the concomitant inhibition of the cell surface carbohydrate binding moiety of agrostin using various monosaccharides. Thus, agrostemmasaponin 1 is able to enhance the transport of agrostin through the cell membrane (possibly by formation of pore-like structures within the phospholipids bilayer) independently of the recognition and binding of cells via the cell-targeting moiety of agrostin. Accordingly, the targeting of specific cells e.g. cancer cells, is not possible.

Accordingly it was an object of the present invention to provide for a composition in which the specific function of a pharmacologically active agent, in particular the cytotoxicity of a chimeric toxin or a toxin conjugate, on target cells is increased. Furthermore it was an object of the present invention to reduce the side-effects associated with the use of pharmacologically active agents, for example during tumor therapy. Furthermore it was an object of the present invention to provide for ways by which the amount of pharmacologically active agents to be used and administered in a therapy can be substantially reduced.

All these objects are solved by a composition comprising at least one pharmacologically active agent coupled to a target cell specific component, and at least one saponin according to claim 1, wherein said pharmacologically active agent is not a saponin, and is not an antigen binding protein comprising an antigen binding region and a region or regions of an antibody that mediate antibody dependent immunological processes. In one embodiment, said pharmacologically active agent is not an agent stimulating an immune response. In a preferred embodiment, said pharmacologically active agent is able to eliminate a dysfunction of a cell or to kill a cell by causing target cell death, preventing target cell proliferation or abolishing or reducing the dysfunction of the target cell by interaction with intracellular targets.

Said pharmacological active agent is cytotoxic or cytostatic.

Said target cell specific component binds specifically to a target cell and is selected from the group comprising (a) proteins, such as natural proteins, recombinant proteins, antibodies, antibody fragments, lipoproteins and protein ligands, such as growth factors (e.g. epidermal growth factor (EGF), granulocyte-macrophage colony-stimulating factor (GM-CSF), vascular endothelial growth factor (VEGF), transforming growth factor (TGF)) and chimeras thereof, cytokines (e.g. interleukins, interferones), transferrin, adhesion molecules (e.g. CD4) and urokinase-type plasminogen activator, (b) peptides, such as natural peptides and synthetic peptides, (c) hormones and combinations of (a) - (c).

Said target cell is selected from the group comprising cancerous cells.

In one embodiment said pharmacologically active agent is coupled to said target cell specific component via covalent linkage.

It is preferred that said target cell specific component is an antibody or an antibody fragment or a growth factor, cytokine or transferrin, or a combination or derivative of any of these.

It is preferred that said target cell specific component binds to a receptor selected form the group comprising growth factor receptors (e.g. HER-1 (epidermal growth factor receptor EGFR), HER-2 HER-3, HER-4 or granulocyte-macrophage colony-stimulating factor receptor GM-CSFR), cytokine receptors (e.g. IL-2R, IL-25R), transferrin receptors, cluster of differentiation (CD) antigens (e.g. CD5, CD6, CD7, CD19, CD22, CD25, CD30, CD33, CD56), Lewis antigens (e.g. Le^{y}) or Wilms' tumor gene product.

Said pharmacologically active agent is a cytotoxin which is selected from the group comprising (a) toxic plant proteins, such as ribosome-inactivating proteins (RIP), in particular type I proteins (e.g. saporin, dianthin, gelonin, PAP) and enzymatically active fragments thereof and the A chain of type II proteins (e.g. from ricin, abrin, mistletoe lectin (viscumin), modeccin) but not excluding other fragments or full length type II proteins, (b) toxic fungal proteins and peptides, such as α-sarcin or mitogillin, (c) bacterial toxins, such as diphtheria toxin or *Pseudomonas* exotoxin, (d) toxic animal proteins, such as angiogenin or granzyme B, in particular of human origin.

Said pharmacologically active agent coupled to said target cell specific component is a chimeric toxin or toxin conjugate, wherein, preferably, said pharmacologically active agent is a peptidic or protein cytotoxin and said target cell specific component is an antibody, growth factor, cytokine or transferrin or a fragment, combination or derivative thereof.

Said pharmacologically active agent coupled to said target cell specific component is a single recombinant protein.

Said saponin is selected from the group comprising triterpenoic saponins with basic structures belonging to the type of 12,13-dehydrooleanane with an aldehyde function in position 23, e.g. Saponinum album from Gypsophila paniculata.

Preferably said chimeric toxin or toxin conjugate is selected from the group comprising Ontak™ (DAB₃₈₉IL2), Zevalin (ibritumomab tituxetan), Mylotarg (gemtuzumab ozogamicin), Saporin-EGF, Herceptin-DM1 (trastuzumab-DM1), RFB4-PE38 (BL22), DAB₃₈₉EGF, H65-RTA, Anti-CD6-bR, Anti-CD7-dgA, TXU-PAP, Anti-Tac-PE38, RFT5-SMPT-dgA, IgG-RFB4-dgA, Di-dgA-RFB4, IgG-HD37-dgA, Anti-B4-bR, Anti-My9-bR, DT388-GM-CSF, B3-LysPE38, B3-PE38, TP40, 454A12-rRA, Tf-CRM107, N901-bR, SGN-15, SGN-25 and SGN-35, Bexxar (tositumomab), Theragyn (pemtumomab). The aforementioned chimeric toxins are described in references (9, 16-19) or are commcercially available from or sponsored by Seragen (Ontak), IDEC Pharmaceuticals (Zevalin), Wyeth Laboratories (Mylotarg), Genentech (Herceptin-DM1), US National Cancer Institute (BL22), Corixa, GlaxoSmith-Kline (Bexxar), Antisoma (Theragyn).

In one embodiment said composition further comprises at least one of the following functional units: a transport protein capable of transporting said pharmacologically active agent into a cell, a cytosolic cleavable bond, preferably a cytosolic cleavable peptide, an endosomal cleavable bond, preferably an endosomal cleavable peptide wherein, preferably, said pharmacologically active agent is coupled to said target cell specific component via said transport protein, and wherein, more preferably, said transport protein is a protein transduction domain (PTD). Such a protein transduction domain has been described above and in references (9-11). Such an entire construct, i.e. comprising a pharmacologically active protein coupled to a target cell specific component via at least one of the following functional units: a protein transduction domain or a cytosolic cleavable peptide or an endosomal cleavable peptide, is herein also sometimes referred to as an "immunoadapter agent", or more specifically "immunoadapter protein".

The objects of the present invention are also solved by the composition according to the present invention for use as a medicament.

Furthermore, the objects of the present invention are also solved by the use of a composition according to the present invention for the manufacture of a medicament for the treatment of an animal having a cancerous disease.

Preferably said saponin is to be administered separately from said pharmacologically active agent coupled to said target cell specific component.

In one embodiment said saponin is to be administered via a different route to the administration route of said pharmacologically active agent coupled to said target cell specific component, wherein, preferably, said saponin is to be administered via injection and said pharmacologically active agent coupled to said target cell specific component is to be administered via ingestion or vice versa.

In another embodiment, said saponin is to be administered via the same route as said pharmacologically active agent coupled to said target cell specific component, wherein, preferably, said administration occurs via injection.

In one embodiment said saponin is to be administered before said pharmacologically active agent coupled to said target cell specific component, wherein, preferably, said saponin is to be administered one minute to one hour, preferably one minute to 45 minutes, more preferably one minute to 30 minutes, most preferably 5 minutes - 20 minutes before administration of said pharmacologically active agent coupled to said target specific component.

In one embodiment said saponin is to be administered at an amount of 100 µg - 100 mg per kg bodyweight of said animal. -

Preferably said animal is a human being.

Furthermore, the objects of the present invention are also solved by a kit comprising, in one or more containers, said composition according to the present invention.

In one embodiment of said kit, said pharmacologically active agent coupled to said target cell specific component, and said saponin are in separate containers.

In one embodiment of said kit, said pharmacologically active agent coupled to said target cell specific component is formulated for injection and said saponin is also formulated for injection.

Preferably, said pharmacologically active agent is able to eliminate a dysfunction of a cell or to kill a cell , in an animal having a disease caused by said dysfunction , by causing target cell death, preventing target cell proliferation or abolishing or reducing the dysfunction of the target cell by interaction of said pharmacological active agent with intracellular targets involved directly or indirectly in said dysfunction, wherein said dysfunction comprises such leading to or being associated with cancer.

Preferably, said pharmacologically active agent is not an agent stimulating an immune response.

In a preferred embodiment said function is a cytotoxic or cytostatic function. Preferably, said disease is selected from the group comprising cancerous diseases.

In one embodiment said pharmacologically active agent coupled to said target cell specific component and said saponin are packaged together, albeit, optionally, in separate containers.

Also disclosed is the composition according to claim 1 for use in a method of treatment of an animal, preferably a human being, having a disease selected from the group comprising cancerous diseases. The administration may occur in any of the ways and order as described before. Preferably said saponin is to be administered before said pharmacologically active agent.

As used herein, the term "pharmacologically active agent" is meant to designate a cytotoxin. The pharmacologically active agent, as used herein, is not a saponin, and is not an antigen binding protein comprising an antigen binding region and a region or regions of an antibody that mediate antibody dependent immunological processes. In one embodiment, it is not an agent stimulating an immune response.

The coupling of said pharmacologically active agent to said target cell specific component may be in the form of a covalent linkage between the two parts or, in the case of the two parts being proteins, a recombinantly produced fusion protein. In one embodiment said pharmacologically active agent coupled to said target cell specific component is a chimeric toxin or a toxin conjugate.

The term "chimeric toxin or a toxin conjugate", as used herein is meant to signify a cytotoxin coupled to an antibody, an antibody fragment or to another component, preferably a ligand, more preferably a protein ligand, such as growth factors, cytokines or transferrin, which recognizes specific structures on a target cell such as growth factor receptors, cytokine receptors, transferrin receptors, cluster of differentiation (CD) antigens, Lewis antigens or Wilms' tumor gene product. It has to be noted, however, that the term "chimeric toxin", as used herein, is not limited to the occurrence of an antibody or antibody fragment therein.

The term "transport protein", as used herein, is meant to designate any protein or peptide that is capable of transporting a pharmacologically active agent into a cell. Examples hereof, such as PTD, have been described above.

The term "immunoadaptertoxin", as used herein, is meant to signify a recombinant chimeric toxin containing in addition to said pharmacologically active agent and said target cell specific component a protein or peptide that contains at least one of the following functional units, a cytosolic cleavable peptide, an endosomal cleavable peptide or a transport protein, such as PTD.

The term "antibody dependent immunological processes", as used herein is meant to designate processes like antibody dependent cellular cytotoxicity, activation of complement, opsonization and phagocytosis.

The term "dysfunction of a cell", as used herein, is meant to designate any deviation of the cell from its normal healthy state. Such dysfunction of a cell may for example manifest itself in a loss of function, loss of differentiation, increased or decreased metabolic turnover, loss of capability of undergoing apoptosis, premature apoptosis, and others.

The inventors have shown that by combining a pharmacologically active agent with a saponin it is possible to enhance the function of the pharmacologically active agent drastically. More specifically, the present inventors' results clearly demonstrate that non-toxic concentrations of saponins, e.g. gypsophilasaponin, are able to enhance the specific cytotoxicity of immunotoxins, e.g. Sap-3 containing ITs (Sap-3 = saporin-3 = type I RIP(ribosome inactivating protein)) dependent on the cell line, 3560 to 385000-fold (Tab. 1). In coming to this conclusion, the present inventors quantitated the specific cytotoxicity of SE, a direct fusion protein of Sap-3 and EGF, and of SA2E, a protein where EGF is linked to Sap-3 via a cleavable molecular adapter that reduces side effects on non-target cells *in vitro* (9). Saponinum album from *Gypsophila paniculata* was used as an example, it is bisdesmosidic and has Gypsogenin as aglycone. Generally speaking, saponins are glycosidic compounds which have one non-sugar component, also termed "aglycone", and one to several sugar residues attached to said aglycone. Saponins having one sugar residue are called monodesmosidic, saponins having two sugar residues attached are termed bisdesmosidic, three sugar residues tridesmosidic etc.. Saponins can be classified according to the type of aglycone they have. The two main types of saponins are steroid saponins and triterpenoic saponins. Many of the triterpenoic saponins are of the oleanane type which is a triterpenoic ring structure having 30 carbon atoms.

In the present inventors' experiments, both ligand-free Sap-3 on target cells as well as complete ITs on receptor-free cells showed a drastically lower cytotoxicity revealing that the effect is receptor- and ligand-specific. The Spn-mediated enhancement of the unspecific toxicity is solely 490 to 1450-fold and independent of the cell line. The high target specificity of saponin-enhanced ITs permits an application of the IT in a broad range and therefore an adjustment to the number of receptors on the target cell. This allows an extremely small dose in the lower picomolar range for tumor cells expressing very high amounts of target receptor (in the present study represented by HER14 cells) as well as higher but still target-specific doses for tumor cells presenting only a weakly elevated number of receptors on their cell surface (represented by MCF-7 cells). Treatment of the latter is only successful with the new combination therapy while sole administration of the IT is not sufficient revealing the high potential of saponin-mediated enhancement. Even at higher concentrations necessary for the treatment of MCF-7 cells, untransfected NIH-3T3 cells (representing »healthy« non-target cells) are not affected.

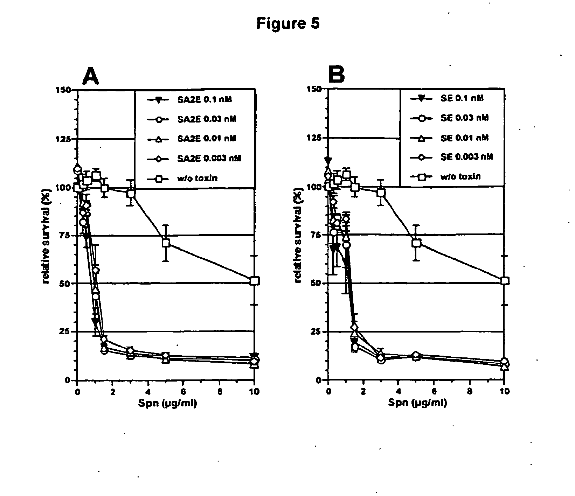

Unlike the variable range of IT being greater than three decimal powers saponin should be applied in a rather narrow range of 1 to 3 µg/ml. The present inventors' results (Fig. 5) revealed that there is a concrete threshold concentration at which the effect of saponin on ITs abruptly sets in. In contrast, the unspecific toxic impact of saponin begins slowly thus reducing the risk of overdosage. Although saponin exerts a toxic effect at high doses, *Quillaja saponaria* Molina (soap bark tree) saponins are orally administered in the form of ISCOMs as adjuvants for vaccination (reviewed in (20)). Moreover, *Quillaja* saponins are a permitted food additive and long-term feeding to rats (0.75 % of total diet) showed no effects on food consumption or body weight (21). Ryokucha saponin, one of the ingredients of green tea *(Camellia sinensis),* had no effect on rats fed for 30 days with 50 mg per kg per day (22). These saponins even show an anti-allergic effect on rats and guinea pigs (23). Furthermore, saponins have not only been tested orally but also intravenously. Consecutive intravenous administrations of ginseng saponins, up to 500 µg per mouse per day, revealed an inhibitory effect on tumor angiogenesis and metastasis but did not generate severe side effects (24, 25). For IT enhancement a dose of approximately 30 µg to a 20 g mouse (weight per weight) corresponding to an optimal saponin concentration (1.5 µg/ml) is sufficient and far below the dose of 500 µg administered by Sato *et al*. (25).

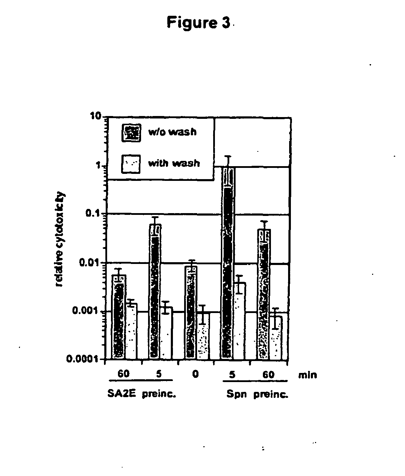

The present invention demonstrates that, surprisingly, the combined administration of individually non-toxic concentrations of an IT and a saponin leads to a highly cytotoxic effect specific for targeted tumor cells and for the first time greater than the cytotoxicity of natural diphtheria toxin for its target cells. The effect is more than additive which was unexpected. When saponins are combined with immunoadaptertoxins instead of conventional ITs further reduction of side effects are likely *in vivo* due to intracellular removal of the ligand and the PTD from the toxin. After cell death the potential of the released toxin entering non-target cells should be drastically reduced since the toxin does not bear a ligand and the enhancer effect of saponins is time-limited (Fig. 3). Moreover, the extremely reduced effective dose can prevent vascular leak syndrome, a dose-dependent side effect of IT therapy characterized by an increase in vascular permeability resulting in interstitial edema and organ failure (7). Other critical side effects resulting from high systemic concentrations and long-term use like severe immune response may also be avoided.

Low required dose, a broad therapeutic concentration window, high cytotoxicity and high specificity advocating the combination therapy presented here offer a new promising tool for tumor therapy.

The invention will now be further described by reference to the following examples. Furthermore, reference is made to the figures wherein the figures show:

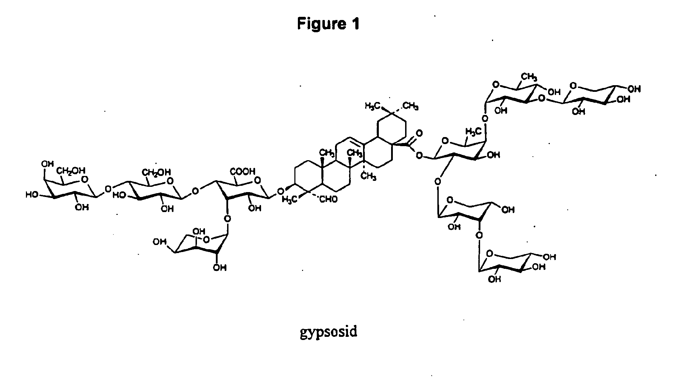

**Fig. 1**. *Structure of the bisdesmosidic triterpenoid saponin gypsosid (Spn), the main component of Saponinum album.* Generally speaking, saponins are glycosidic compounds which have one non-sugar component, also termed "aglycone", and one to several sugar residues attached to said aglycone. Saponins having one sugar residue are called monodesmosidic, saponins having two sugar residues attached are termed bisdesmosidic, three sugar residues tridesmosidic etc.. Saponins can be classified according to the type of aglycone they have. The two main types of saponins are steroid saponins and triterpenoic saponins. Many of the triterpenoic saponins are of the oleanane type which is a triterpenoic ring structure having 30 carbon atoms.

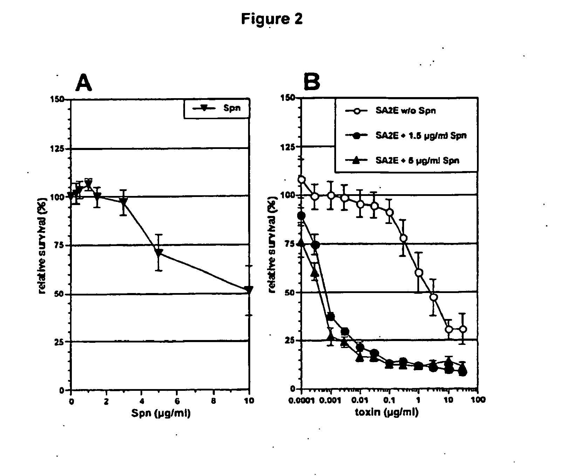

**Fig. 2****.** *Cytotoxicity of Spn alone and its effect on the toxicity of the immunoadaptertoxin SA2E*. **A:** HER14 cells were incubated for 48 h in the presence of varying concentrations of Spn. Hereafter, living cells were quantitated by their ability to cleave fluorescein diacetate. Relative survival was calculated as the number of living cells after treatment in relation to untreated cells. Error bars indicate the standard error of the mean (SEM) of 4 experiments. B: HER14 cells were incubated at varying concentrations of the immunoadaptertoxin SA2E consisting of Sap-3, a PDT-containing molecular adapter and EGF as ligand. Incubation was performed in the absence of Spn (open circle) and presence of 1.5 µg/ml (closed circle) and 5 µg/ml (closed triangle). Relative survival was calculated as described in A. Error bars: SEM of 7 experiments.
**Fig. 3****.** *Effect of order, washout and preincubation periods on cytotoxicity*. HER14 cells were preincubated with either varying concentrations of the immunoadaptertoxin SA2E (two left column pairs) or 1.5 µg/ml Spn (two right column pairs) for 5 or 60 min. After preincubation 1.5 µg/ml Spn (two left column pairs) or different concentrations of SA2E (two right column pairs) were added after washout of the preincubated compound (light columns) or added directly (dark columns), incubated for 48 h and the IC₅₀ for each preincubation time determined. The central columns (0 min) show concomitant administration of Spn and SA2E (dark column) and incubation of SA2E alone (light column). The column height represents the IC₅₀ relative to the sample with 5 min pretreatment of Spn and error bars the SEM of 4 experiments (8 experiments for 5 min pretreatment of Spn). Take note of the logarithmic scale.

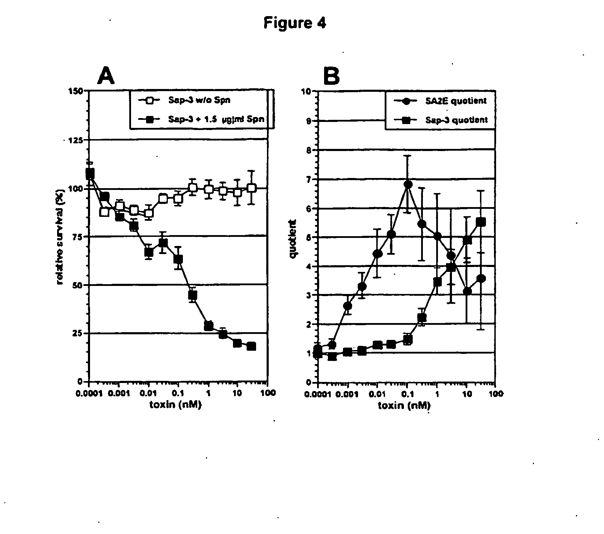

**Fig. 4****.** *Ligand-independent cytotoxicity in the presence and absence of Spn*. **A:** HER14 cells were treated with varying concentrations of ligand-free Sap-3 in the absence (open squares) and presence of 1.5 µg/ml Spn (closed squares). The relative survival after 48 h is displayed with SEM of 10 experiments. **B:** To visualize the cytotoxic enhancement the quotient of the relative survival in the absence and presence of Spn for the immunoadaptertoxin SA2E (closed circles) and ligand-free Sap-3 (closed squares) is depicted.
**Fig. 5****.** *Effect of different Spn concentrations on adapter-containing and adapter-free immunotoxins.* **A:** HER14 cells were incubated for 48 h with SA2E in four different concentrations together with varying concentrations of Spn and the relative survival determined. **B:** Instead of SA2E the adapter-free immunotoxin SE (Sap-3 directly linked to EGF) was applied. In both panels the curve for the unspecific effect of Spn alone already displayed in Fig. 2A was added for comparison. All error bars represent the SEM of 4 experiments (7 experiments for Spn alone).

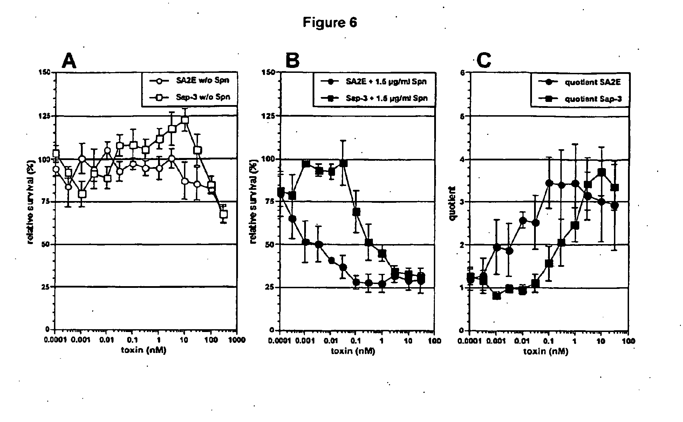

**Fig. 6****.** *Receptor-dependent cytotoxicity in the presence and absence of Spn*. Displayed is the relative survival of MCF-7 cells, expressing about 40 times less EGFR on their cell surface compared to HER14 cells, after incubation for 48 h with varying concentrations of SA2E (circles) or ligand-free Sap-3 (squares) in the absence (**A**, open symbols) and presence (**B**, closed symbols) of 1.5 µg/ml Spn. Error bars: SEM of 4 to 6 experiments. **C**: Quotient of the relative survival in the absence and presence of Spn for SA2E (circles) and Sap-3 (squares).

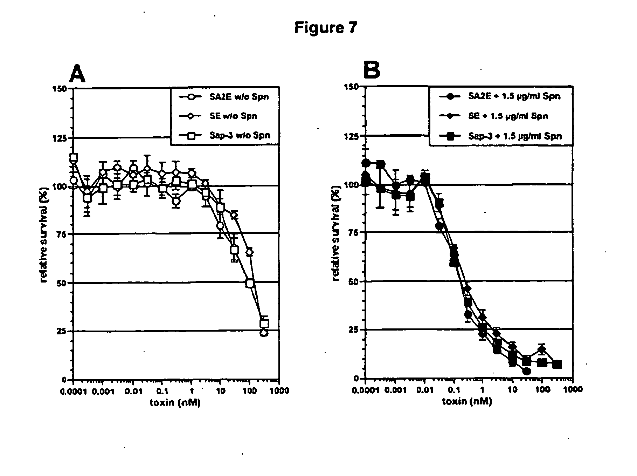

**Fig. 7****.** *Receptor-independent cytotoxicity in the presence and absence of Spn.* **A:** Relative survival of NIH-3T3 cells, expressing no human EGFR, after incubation for 48 h with varying concentrations of SA2E (circles), SE (rhombs) or ligand-free Sap-3 (squares) in the absence of Spn. **B:** Same as in A but in the presence of 1.5 µg/ml Spn. Error bars in both panels: SEM of 4 experiments.

### Example 1

Abbreviations: EGF, epidermal growth factor; EGFR, EGF receptor; IT, immunotoxin; PBS, phosphate-buffered saline; PTD, protein transduction domain; RIP, ribosome-inactivating protein; Sap-3, His-tagged saporin-3; Spn, Saponinum album from *Gypsophila paniculata* L*;* SE, direct fusion protein of Sap-3 and EGF; SA2E, EGF linked to Sap-3 via a cleavable molecular adapter.

### Plasmid construction and immunoadaptertoxin expression

All primers and oligonucleotides were purchased from Metabion (Martinsried, Germany). The DNA of the IT was constructed in pLitmus28 (NEB, Frankfurt, Germany) and transcribed from a pET11d expression vector (Novabiochem, Schwalbach, Germany) as previously described in detail (9). The immunoadaptertoxin used in this study (SA2E, identical to SapAd*EGF in (9)) consists of an N-terminal 6x His-tag, saporin-3 (this cDNA was generously provided by Serena Fabbrini, San Raffaele Scientific Institute, Milano, Italy), the adapter and EGF. The adapter is composed of a cytosolic cleavable peptide (YVHDEVDRGP) containing caspase cleavage sites and a yeast recognition sequence for cytosolic cleavage, a PTD (PLSSIFSRIGDP) derived from the PreS2-domain of hepatitis B virus surface antigen (26) and an endosomal cleavable peptide (RHRQPRGNRVGRS) containing the *Pseudomonas* exotoxin and a modified diphtheria toxin cleavage site. As a control for ligand-mediated specificity His-tagged saporin-3 (Sap-3) without adapter and ligand was used.

For expression, plasmid DNA was transformed into the *Escherichia coli* strain Rosetta DE3 pLysS (Novagen, Schwalbach, Germany). Transformed cells were grown overnight in LB medium supplemented with 50 µg/ml ampicillin and 0.5% glucose. The overnight culture was used to inoculate 1 liter LB medium containing 50 µg/ml ampicillin and grown to A₆₀₀ = 0.5-0.6. Isopropyl β-D-thiogalactopyranoside was added to a final concentration of 1 mM and the culture further incubated for 3 h. Cells were harvested by centrifugation (10 min, 4°C, 3400 g) and the culture pellets were either stored at -20°C or processed immediately.

### Purification of the immunoadaptertoxin and testing of the enzymatic activity

The pellets were lysed under denaturing conditions and Sap-3 as well as SA2E purified by nickel-nitrilotriacetic acid agarose chromatography according to the manufacturers' recommendations (Qiagen, Hilden, Germany). Briefly, the cells were sonicated on ice three times for 30 s (Branson sonoplus, microtip SH213G, duty cycle 20%) with 1 min breaks and centrifuged at 48000 g for 30 min at 4°C. The supernatant was subjected to affinity chromatography on a 4 ml pre-equilibrated nickel-nitrilotriacetic acid column. The toxins were eluted using 8 M urea (pH 4.5) and the corresponding fractions dialyzed against 50 mM Tris-HCl / 100 mM NaCl (pH 7.5). The final materials were estimated to be 95% pure as evaluated by Coomassie and silver staining after SDS-PAGE.

The enzymatic activity of saporin-3 in all constructs was quantified using a colorimetric assay as described (27). All toxins used in the present study exhibited *in vitro* an identical N-glycosidase activity on nucleic acid substrates. The concentration of purified protein was estimated using Advanced Protein Assay Kit (Cytoskeleton Inc.). Quantity of full-length toxins was corrected after analyses by SDS-PAGE [12% (w/v) gel] under reducing conditions and densitometric scanning using protein standards.

### Cell Culture experiments

Cell culture experiments were performed with untransfected Swiss mouse embryo NIH-3T3 cells (obtained from DSMZ, the German Collection of Microorganisms and Cell Cultures) as control, NIH-3T3 cells transfected with human EGF receptor (EGFR) referred to as HER14 cells (a kind gift from Prof. E. J. van Zoelen, Department of Cell Biology, University of Nijmegen, The Netherlands), and the human breast adeno-carcinoma cell line MCF-7 (obtained from ATCC, Rockville, MD). Whereas HER14 cells express about 4 × 10⁵ EGFR molecules per cell MCF-7 cells possess a lower number of approximately 1 × 10⁴ EGFR per cell. Cells were maintained in Dulbecco's MEM with Glutamax™ 1 (Invitrogen /Gibco, Karlsruhe, Germany) supplemented with 10% FCS (BioChrom KG, Berlin, Germany), 100 U/ml penicillin and 100 µg/ml streptomycin. Cells were cultivated at 37°C, 5% CO₂ and 95% humidity.

For the determination of the dose response curves, the cells were trypsinated, washed 3x with PBS, seeded in 96-well plates (pretreated with 0.1% gelatin in PBS) at a concentration of 5000 cells per well and left untreated for 16 h. The cells were washed twice with PBS and incubated with 180 µl medium containing 1.5 µg/ml Saponinum album from *Gypsophila paniculata* L. (Spn) except when varied as indicated in the figures. Spn was purchased from Merck AG, Germany. After 5 min 20 µl toxin (Sap-3 or SA2E) was added to defined final concentrations generally in the range of 0.0001 to 30 nM and the cells cultivated for 48 h. To determine the effect of synergistic cytotoxicity in dependence on the time and order of application, Spn was preincubated for 5 min or 1 h and then either washed out by tandem wash with PBS or left on the cells together with the toxins. In analogy the toxins were preincubated for 5 min or 1 h and then either washed out or incubated together with Spn (added from a stock solution of 3 mg/ml). When applied at the same time, Sap-3 and Spn were premixed and added to 180 µl medium.

### Cytotoxicity assay

The cytotoxicity assay is based on the cleavage of fluorescein diacetate by living cells and was performed as described by Nygren *et al*. (28). After incubation of the cells with the toxins, they were washed twice with PBS and incubated with fluorescein diacetate (10 µg/ml; Sigma, Germany) for 1 h. Fluorescence was measured using a microplate reader (Spectra Max Gemini, Molecular Devices) with an excitation and emission λ of 485 nm and 538 nm respectively. The survival index was calculated after blank subtraction (wells without cells) as the percentage of living cells in treated wells in relation to untreated cells (cells without toxin).

### Example 2

### Optimization of concentration and point of time for Spn administration

The purification, testing of the enzymatic activity and determination of dose response curves is well established for the immunoadaptertoxins (9, 27). For all experiments only those toxins were used that exhibit at least 95% purity (as evaluated by Coomassie and silver staining) and an adenine release from herring sperm DNA in the range of 600-700 pmol adenine per pmol toxin per hour (as determined by a colorimetric adenine quantification assay (27)). Spn (Fig. 1) was first tested for its cytotoxicity either alone or in two selected concentrations in the presence of the immunoadaptertoxin SA2E on target receptor-expressing HER14 cells. Spn has no or, if any, a minute stimulating effect on HER14 cells up to 3 µg/ml (Fig. 2A). At 5 µg/ml and higher concentrations a considerable cytotoxic effect occurred (IC₅₀ = 10.3 µg/ml, extrapolated). Applying a non-toxic (1.5 µg) and a toxic concentration (5 µg) of Spn in a SA2E dose response study (Fig. 2B) clearly shows that Spn at the non-toxic concentration enhances the cytotoxicity of SA2E about 3560-fold (from an IC₅₀ of 2.4 nM to 0.67 pM). In contrast, the effect of a further increase in the Spn concentration to the toxic level of 5 µg/ml only increases the cytotoxicity to an IC₅₀ of 0.44 pM indicating that the extreme synergistic effect is mediated at non-toxic concentrations of Spn whereas the additional effect of toxic Spn concentrations is solely additive. The same relative enhancement of the toxic effect mediated by Spn at 5 µg/ml compared to 1.5 µg/ml is also observed (at absolute higher concentrations) when applying ligand-free Sap-3 indicating that this effect is ligand-independent and therefore undesirable. Thus, the present inventors used 1.5 µg/ml Spn as standard for all further investigations. Nevertheless, the present inventors also performed most experiments with 5 µg/ml Spn, the effect, however, does not differ from the effect shown in Fig. 2B and is therefore not shown for succeeding data (except for explicit Spn variations).

Since the mechanism of synergism is unknown it was unclear whether a parallel premixed or a subsequent administration is advantageous and, if the latter is true, which compound should be applied first and for which time period. The present inventors performed all corresponding experiments in two modes, namely with washout of the first component before adding the second and simply adding the second component resulting in a 48-h-incubation of both SA2E and Spn. Fig. 3 shows that a washout of either component led to no enhanced cytotoxicity compared to SA2E treatment alone (0 min, light column), except when cells were pretreated with Spn for 5 min. The lack of synergism after washout indicates that Spn action on cells is reversible (as shown by Spn preincubation) and that Spn must be present before SA2E binding to its receptor (as shown by SA2E preincubation). In comparison without washout, preincubation for 5 min with SA2E results in an enhanced synergism, which is reduced to the level without preincubation (0 min, dark column) after a longer preincubation period. This may be attributed to the internalization of bound SA2E preventing Spn from colocalizing. Pretreatment of the cells with Spn led to a higher cytotoxicity than in the corresponding experiments with SA2E pretreatment. Similar to SA2E preincubation, 60 min preincubation with Spn resulted in a decreased synergistic effect compared to 5 min preincubation, however, this cytotoxicity is still greater than that of without preincubation. This implies that an interaction of Spn with the cell membrane prepares the cell for improved uptake. The decrease with longer incubation times is possibly due to metabolic degradation or oxidation of saponins. Surprisingly, a simultaneous treatment with Spn and SA2E causes a substantially lower cytotoxicity than short pretreatment with either of the two compounds. Without wishing to be bound by any theory, this suggests that Spn and SA2E interact directly in a way mutually preventing them from fulfilling their task. The maximum synergistic toxicity is reached when Spn is preincubated for 5 min and SA2E added without washing out Spn, thus these conditions were applied for all further experiments.

### Effects of Spn on the ligand-dependent specificity

To investigate how Spn affects the specificity of ITs two series of experiments were performed. First, the effect on the cytotoxicity of SA2E and ligand-free Sap-3 was measured to determine the role of the ligand; second, SA2E was tested on different cell lines that vary in the cell surface expression of EGFR (see below) to define the role of the receptor. Ligand-free Sap-3 in the absence of Spn revealed no cytotoxicity up to 30 nM (Fig. 4A) and an IC₅₀ of 175 nM (IC₅₀ not shown). In contrast, in the presence of the non-toxic concentration of Spn (1.5 µg) Sap-3 exhibited a 790-fold higher cytotoxic effect with an IC₅₀ of 0.22 nM (Fig. 4A). The observed cytotoxicity is 10 times higher than that of SA2E in the absence of Spn (2.4 nM), however, 330-fold less than that of SA2E in the presence of Spn (0.67 pM). Thus the enhancer effect of Spn on the unspecific toxicity of ligand-free Sap-3 is 5-fold lower than the enhancer effect on target-cell specific SA2E (compare 790-fold for Sap-3 with 3560-fold for SA2E, see Tab. 1). This means that the combined application of Spn with SA2E broadens the therapeutic concentration window of SA2E and simultaneously drastically decreases the concentration required for effective target-cell treatment.

To better quantitate and visualize these effects the present inventors calculated the quotient of the relative cytotoxicities determined in the presence and absence of Spn. Whereas a quotient near to one at low SA2E concentrations means that under both conditions (with and without Spn) no toxic activity can be observed, the decline of the quotient at higher concentrations is attributed to increasing cytotoxicity of SA2E in the absence of Spn. At the peak concentration the enhancer effect of Spn reaches its maximum. Fig. 4B reveals that Spn shows clear effects on SA2E cytotoxicity at IT concentrations greater than 0.3 pM with a maximum at 100 pM. In contrast an impact on Sap-3 cytotoxicity is only observed at concentrations greater than 100 pM. Thus a very broad therapeutic window exists ranging from 1 to 100 pM of the IT.

To ensure that the observed effect of Spn is truly ligand-dependent and does not depend on the molecular adapter the present inventors also tested an adapter-free IT where EGF is directly coupled to Sap-3 (SE). Comparing the cytotoxicity at different Spn concentrations for 4 IT concentrations within the therapeutic window reveals that no significant difference can be observed for SA2E and adapter-free SE (Fig. 5A and 5B respectively). The experiments presented here corroborate our preceding results which showed that the ITs exhibit similar cytotoxicity and that the reduced side-effects of the immunoadaptertoxins on non-target cells are ascribed to their desired intracellular cleavage inside the target cells and reduced half-life (9). The present inventors performed all other investigations in the present study, in addition to SA2E, also with SE. The results were nearly identical and thus exemplarily shown in addition only in Fig. 7. Independent of the IT concentration all curves in Fig. 5 exhibit nearly the same course indicating that all concentrations lie within the therapeutic window. This broad window can be advantageously utilized to adjust the IT concentration to the requirements of the target cell (see below).

### Effects of Spn on receptor-dependent specificity

To investigate receptor-dependent specificity, the present inventors analyzed the Spn-mediated effect on the cytotoxicity of SA2E and Sap-3 additionally in MCF-7 cells and untransfected NIH-3T3 cells. The human breast adeno-carcinoma cell line MCF-7 expresses about 40-fold less EGFR molecules than HER14 (1 × 10⁴ receptors per cell compared to 4 × 10⁵). In contrast to the toxicity on HER14 cells SA2E only exhibits a slight cytotoxic effect on MCF-7 cells not reaching the IC₅₀ at 300 nM (Fig. 6A). This corresponds to previous results and is probably due to the lower receptor number. In the presence of Spn, however, a clear cytotoxic effect with an IC₅₀ of about 2.7 pM is observed, a concentration 4-fold higher than for HER14 cells (0.67 pM, Fig. 2B) and thus mirroring the reduced receptor number on MCF-7 cells. Although Sap-3 does not possess a ligand and thus cytotoxicity is independent of the receptor level on the cell surface it has a slightly lower effect on MCF-7 cells (0.63 nM compared to 0.22 nM on HER14). Since HER14 and MCF-7 cells are completely different cell lines from mouse and human, respectively, they apparently exhibit a different metabolic sensitivity against Sap-3. When extrapolating the cytotoxicity curve in Fig. 6A for MCF-7 cells incubated without Spn an IC₅₀ of about 1000 nM is calculated for both SA2E and Sap-3 (Tab. 1). Thus, Spn enhances the unspecific ligand-independent cytotoxicity of Sap-3 for MCF-7 cells only 1450-fold, in contrast, the specific ligand-dependent cytotoxicity of SA2E 385000-fold. The quotients of the treatments with and without Spn showed an optimal dose in the range of 0.01 to 0.3 nM (Fig. 6C) demonstrating that the effective IT concentration can be easily adjusted according to the receptor expression on the surface of the target cells. The higher scattering of the values is due to slightly raised scattering of the primary data that is strengthened by quotient formation.

When applied to human EGFR-free NIH-3T3 cells no difference is observed between Sap-3, SA2E and SE (Fig. 7A, B) demonstrating that the specific uptake of SA2E and SE is strongly receptor-dependent. The identical curves mirror the unspecific uptake of the ITs. The IC₅₀ was calculated to 83 (Sap-3), 112 (SE) and 135 nM (SA2E, extrapolated) in the absence of Spn and to 0.17 (Sap-3), 0.13 (SA2E) and 0.25 nM (SE) in the presence of Spn. These values are nearly identical to those determined for Sap-3 on HER14 cells (0.22 nM), which was expected since HER14 cells are transfected NIH-3T3 cells.

The present inventors' results clearly show that the effective concentrations of pharmacologically active agents, e.g. ITs can be drastically reduced when administered in combination with non-toxic concentrations of a saponin, e.g. Spn. Moreover the therapeutic concentration window is broadened since the enhancer effect of the saponin on the pharmacologically active agent without a target cell specific component, e.g. a ligand, is strikingly less than on those agents with a target cell specific component, e.g. ITs. Thus, the concept of combined application of a saponin and a pharmacologically active agent coupled to a target cell specific component is a promising tool for enhancing the function of the pharmacologically active agent, as exemplified by the improved specific cytotoxicity of ITs on target cells and reduced side-effects during tumor therapy.

### REFERENCES

1. Brinkmann, U. 2000. Recombinant antibody fragments and immunotoxin fusions for cancer therapy. In Vivo. 14:21-27.
2. LeMaistre, C.F., Saleh, M.N., Kuzel, T.M., Foss, F., Platanias, L.C., Schwartz, G., Ratain, M., Rook, A., Freytes, C.O., Craig, F. et al. 1998. Phase I trial of a ligand fusion-protein (DAB389IL-2) in lymphomas expressing the receptor for interleukin-2. Blood. 91:399-405.
3. Piascik, P. 1999. FDA approves fusion protein for treatment of lymphoma. J Am Pharm Assoc (Wash). 39:571-572.
4. Wiseman, G.A., White, C.A., Sparks, R.B., Erwin, W.D., Podoloff, D.A., Lamonica, D., Bartlett, N.L., Parker, J.A., Dunn, W.L., Spies, S.M. et al. 2001. Biodistribution and dosimetry results from a phase III prospectively randomized controlled trial of Zevalin radioimmunotherapy for low-grade, follicular, or transformed B-cell non-Hodgkin's lymphoma. Crit Rev Oncol Hematol. 39:181-194.
5. Wiseman, G.A., White, C.A., Stabin, M., Dunn, W.L., Erwin, W., Dahlbom, M., Raubitschek, A., Karvelis, K., Schultheiss, T., Witzig, T.E. et al. 2000. Phase I/II 90Y-Zevalin (yttrium-90 ibritumomab tiuxetan, IDEC-Y2B8) radioimmunotherapy dosimetry results in relapsed or refractory non-Hodgkin's lymphoma. Eur J Nucl Med. 27:766-777.
6. Hamann, P.R., Hinman, L.M., Hollander, I., Beyer, C.F., Lindh, D., Holcomb, R., Hallett, W., Tsou, H.R., Upeslacis, J., Shochat, D. et al. 2002. Gemtuzumab ozogamicin, a potent and selective anti-CD33 antibody-calicheamicin conjugate for treatment of acute myeloid leukemia. Bioconjug Chem. 13:47-58.
7. Vitetta, E.S. 2000. Immunotoxins and vascular leak syndrome. Cancer J Sci Am. 6 Suppl 3:S218-224.
8. Keller, J., Heisler, I., Tauber, R., and Fuchs, H. 2001. Development of a novel molecular adapter for the optimization of immunotoxins. J Control Release. 74:259-261.
9. Heisler, I., Keller, J., Tauber, R., Sutherland, M., and Fuchs, H. 2003. A cleavable adapter to reduce nonspecific cytotoxicity of recombinant immunotoxins. Int J Cancer. 103:277-282.
10. Fischer, P.M., Krausz, E., and Lane, D.P. 2001. Cellular delivery of impermeable effector molecules in the form of conjugates with peptides capable of mediating membrane translocation. Bioconjug Chem. 12:825-841.
11. Beerens, A.M., Al Hadithy, A.F., Rots, M.G., and Haisma, H.J. 2003. Protein transduction domains and their utility in gene therapy. Curr Gene Ther. 3:486-494.
12. Engert, A., Brown, A., and Thorpe, P. 1991. Resistance of myeloid leukaemia cell lines to ricin A-chain immunotoxins. Leuk. Res. 15:1079-1086.
13. Brusa, P., Dosio, F., Coppo, S., Pacchioni, D., Arpicco, S., Crosasso, P., and Cattel, L. 1997. In vitro and in vivo antitumor activity of immunoconjugates prepared by linking 5-fluorouridine to antiadenocarcinoma monoclonal antibody. Farmaco. 52:71-81.
14. Kreitman, R.J. 2001. Toxin-labeled monoclonal antibodies. Curr Pharm Biotechnol. 2:313-325.
15. Falnes, P.O., Wesche, J., and Olsnes, S. 2001. Ability of the Tat basic domain and VP22 to mediate cell binding, but not membrane translocation of the diphtheria toxin A-fragment. Biochemistry. 40:4349-4358.
16. Allen, T.M. 2002. Ligand-targeted therapeutics in anticancer therapy. Nat Rev Cancer. 2:750-763.
17. Carter, P. 2001. Improving the efficacy of antibody-based cancer therapies. Nat Rev Cancer. 1:118-129.
18. Payne, G. 2003. Progress in immunoconjugate cancer therapeutics. Cancer Cell. 3:207-212.
19. Kreitman, R.J. 1999. Immunotoxins in cancer therapy. Curr Opin Immunol. 11:570-578.
20. Sjölander, A., and Cox, J.C. 1998. Uptake and adjuvant activity of orally delivered saponin and ISCOM vaccines. Adv Drug Deliv Rev. 34:321-338.
21. Rao, A.V., and Kendall, C.W. 1986. Dietary saponins and serum lipids. Food Chem Toxicol. 24:441.
22. Kawaguchi, M., Kato, T., Kamada, S., and Yahata, A. 1994. Three-month oral repeated administration toxicity study of seed saponins of Thea sinensis L. (Ryokucha saponin) in rats. Food Chem Toxicol. 32:431-442.
23. Akagi, M., Fukuishi, N., Kan, T., Sagesaka, Y.M., and Akagi, R. 1997. Anti-allergic effect of tea-leaf saponin (TLS) from tea leaves (Camellia sinensis var. sinensis). Biol Pharm Bull. 20:565-567.
24. Mochizuki, M., Yoo, Y.C., Matsuzawa, K., Sato, K., Saiki, L, Tono-oka, S., Samukawa, K., and Azuma, I. 1995. Inhibitory effect of tumor metastasis in mice by saponins, ginsenoside-Rb2, 20(R)- and 20(S)-ginsenoside-Rg3, of red ginseng. Biol Pharm Bull. 18:1197-1202.
25. Sato, K., Mochizuki, M., Saiki, I., Yoo, Y.C., Samukawa, K., and Azuma, I. 1994. Inhibition of tumor angiogenesis and metastasis by a saponin of Panax ginseng, ginsenoside-Rb2. Biol Pharm Bull. 17:635-639.
26. Oess, S., and Hildt, E. 2000. Novel cell permeable motif derived from the PreS2-domain of hepatitis-B virus surface antigens. Gene Ther. 7:750-758.
27. Heisler, I., Keller, J., Tauber, R., Sutherland, M., and Fuchs, H. 2002. A Colorimetric Assay for the Quantitation of Free Adenine Applied to Determine the Enzymatic Activity of Ribosome-Inactivating Proteins. Anal Biochem. 302:114-122.
28. Nygren, P., Fridborg, H., Csoka, K., Sundstrom, C., de la Torre, M., Kristensen, J., Bergh, J., Hagberg, H., Glimelius, B., Rastad, J. et al. 1994. Detection of tumor-specific cytotoxic drug activity in vitro using the fluorometric microculture cytotoxicity assay and primary cultures of tumor cells from patients. Int J Cancer. 56:715-720.
**29.** Hebestreit, P. and Melzig, M.F. 2003. Cytotoxic Activity of the Seeds from Agrostemma githago var. githago. Planta Med. 69:921-925**.**

### TABLES

**Tab. 1. IC₅₀ values (nM) and Spn-mediated factors of enhancement for Sap-3 and SA2E on different cell lines in the absence and presence of Spn.**

| **cell line** | **Sap-3** | | | **SA2E** | | |
|---|---|---|---|---|---|---|
| | without Spn | 1.5 µg/ml Spn | factor of enhancement | without Spn | 1.5 µg/ml Spn | factor of enhancement |
| HER14 | 175 | 0.22 | 790 | 2.4 | 0.00067 | 3560 |
| MCF-7 | 920 ex | 0.63 | 1450 | 1040 ex | 0.0027 | 385000 |
| NIH-3T3 | 83 | 0.17 | 490 | 135 ex | 0.13 | 1050 |

| | | | | | | |
|---|---|---|---|---|---|---|
| ex: values are extrapolated from the data in Fig. 6A and 7A | | | | | | |

## Claims

1. A composition comprising
at least one pharmacologically active agent which has a detectable pharmacological effect on a cell or an organism, coupled to a target cell specific component that binds specifically to a target cell, and
at least one saponin, said saponin being selected from the group comprising triterpenoic saponins with basic structures belonging to the type of 12,13-dehydrooleanane with an aldehyde function in position 23,
wherein said pharmacologically active agent coupled to said target cell specific component is a chimeric toxin or a toxin conjugate, said chimeric toxin or toxin conjugate being a single recombinant protein,
wherein said pharmacologically active agent is not a saponin, and is not an antigen binding protein comprising an antigen binding region and a region or regions of an antibody that mediate antibody dependent immunological processes, such as antibody dependent cellular cytotoxicity, activation of complement, opsonization and phagocytosis, wherein said target cell specific component is selected from the group comprising (a) proteins, such as natural proteins, recombinant proteins, antibodies, antibody fragments, lipoproteins and protein ligands, such as growth factors e.g. EGF, GM-CSF, VEGF, TGF and chimeras thereof, cytokines e.g. interleukins, interferones, transferrin, adhesion molecules e.g. CD4 and urokinase-type plasminogen activator, (b) peptides, such as natural peptides and synthetic peptides, (c) hormones and combinations of (a) - (c), and wherein said target cell is selected from the group comprising cancerous cells, and wherein said pharmacologically active agent is a cytotoxin which is selected from the group comprising (a) toxic plant proteins, such as ribosome-inactivating proteins (RIP), in particular type I proteins e.g. saporin, dianthin, gelonin, PAP and enzymatically active fragments thereof and the A chain of type II proteins e.g. from ricin, abrin, mistletoe lectin (viscumin), modeccin but not excluding other fragments or full length type II proteins, (b) toxic fungal proteins and peptides, such as α-sarcin or mitogillin, (c) bacterial toxins, such as diphtheria toxin or *Pseudomonas* exotoxin, and (d) toxic animal proteins, such as angiogenin or granzyme B, in particular of human origin.

2. The composition according to claim 1, **characterized in that**, in said chimeric toxin or toxin conjugate, said pharmacologically active agent is coupled to said target cell specific component via covalent linkage.

3. The composition according to any of the foregoing claims, **characterized in that** said target cell specific component is an antibody or an antibody fragment or a growth factor, cytokine or transferrin or a combination of any of these.

4. The composition according to any of the foregoing claims, wherein said saponin is Saponinum album from Gypsophila paniculata.

5. The composition according to any of the foregoing claims, **characterized in that** said composition further comprises at least one of the following functional units: a transport protein capable of transporting said pharmacologically active agent into a cell, a cytosolic cleavable bond, an endosomal cleavable bond.

6. The composition according to claim 5, **characterized in that** said pharmacologically active agent is coupled to said target cell specific component via said transport protein, cytosolic cleavable bond and/or endosomal cleavable bond.

7. The composition according to any claims 5 - 6, **characterized in that** said transport protein is a protein transduction domain (PTD), said cytosolic cleavable bond is part of a peptide and said endosomal cleavable bond is part of a peptide.

8. The composition according to any of the foregoing claims for use as a medicament.

9. Use of a composition according to any of the foregoing claims for the manufacture of a medicament for the treatment of an animal having a disease selected from the group comprising cancerous diseases.

10. Use according to claim 9, **characterized in that** said saponin is to be administered separately from said pharmacologically active agent coupled to said target cell specific component.

11. Use according to any of claims 9 - 10, **characterized in that** said saponin is to be administered via a different route to the administration route of said pharmacologically active agent coupled to said target cell specific component.

12. Use according to claim 11, **characterized in that** said saponin is to be administered via inject tion and said pharmacologically active agent coupled to said target cell specific component is administered via ingestion or vice versa.

13. Use according to any of claims 9 - 10, **characterized in that** said saponin is to be administered via the same route as said pharmacologically active agent coupled to said target cell specific component.

14. Use according to claim 13, **characterized in that** said administration occurs via injection.

15. Use according to any of claims 9 - 15, **characterized in that** said saponin is to be administered before said pharmacologically active agent coupled to said target cell specific component.

16. A kit comprising, in one or more containers, said composition according to any of claims 1 - 8.

17. Kit according to claim 16, **characterized in that** said pharmacologically active agent coupled to said target cell specific component, and said saponin are in separate containers.

## Patentansprüche

1. Zusammensetzung, umfassend
wenigstens ein pharmakologisch aktives Mittel, das einen nachweisbaren pharmakologischen Effekt auf eine Zelle oder einen Organismus hat, gekoppelt an einen Zielzell-spezifischen Bestandteil, der spezifisch an eine Zielzelle bildet, und
wenigstens ein Saponin, wobei das Saponin ausgewählt ist aus der Gruppe, umfassend triterpenoide Saponine mit einer Grundstruktur vom Typ des 12,13-Dehydrooleanan mit einer Aldehydfunktion in Position 23,
wobei das pharmakologisch aktive Mittel, gekoppelt an den Zielzell-spezifischen Bestandteil, ein chimäres Toxin oder ein Toxin-Konjugat ist, wobei das chimäre Toxin oder Toxin-Konjugat ein einzelnes rekombinantes Protein ist,
wobei das pharmakologisch aktive Mittel kein Saponin ist und kein Antigen-bindendes Protein ist, umfassend eine Antigen-bindende Region und eine Region oder Regionen eines Antikörpers, die Antikörper-abhängige immunologische Prozesse vermitteln, wie etwa Antikörper-abhängige zelluläre Zytotoxizität, Komplementaktivierung, Opsonisierung und Phagozytose, wobei der Zielzell-spezifische Bestandteil ausgewählt ist aus der Gruppe, umfassend (a) Proteine, wie etwa natürliche Proteine, rekombinante Proteine, Antikörper, Antikörper-Fragmente, Lipoproteine und Proteinliganden, wie etwa Wachstumsfaktoren, z.B. EGF, GM-CSF, VEGF, TGF und Chimären davon, Zytokine, z.B. Interleukine, Interferone, Transferrin, Adhäsionsmoleküle, z.B. CD4, und Urokinase-Typ Plasminogen-Aktivator, (b) Peptide, wie etwa natürliche Peptide und synthetische Peptide, (c) Hormone und Kombinationen von (a) - (c), und wobei die Zielzelle ausgewählt ist aus der Gruppe, umfassend Krebszellen, und wobei das pharmakologische aktive Mittel ein Zytotoxin ist, das ausgewählt ist aus der Gruppe, umfassend (a) toxische Pflanzenproteine, wie etwa Ribosomen-inaktivierende Proteine (RIP), insbesondere Proteine vom Typ I, z.B. Saporin, Dianthin, Gelonin, PAP und enzymatisch aktive Fragmente davon, und die A-Kette von Proteinen des Typs II, z.B. von Ricin, Abrin, Mistel-Lectin (Viscumin), Modeccin, aber nicht ausschließlich anderer Fragmente oder Vollängenproteine vom Typ II, (b) toxische Pilzproteine und Peptide, wie etwa α-Sarcin oder Mitogillin, (c) bakterielle Toxine, wie etwa Diphtherie-Toxin oder Pseudomonas-Exotoxin, und (d) toxische Tierproteine, wie etwa Angiogenin oder Granzym B, insbesondere menschlichen Ursprungs.

2. Zusammensetzung nach Anspruch 1, **dadurch gekennzeichnet, dass** in dem chimären Toxin oder Toxin-Konjugat das pharmakologisch aktive Mittel an den Zielzell-spezifischen Bestandteil über eine kovalente Verknüpfung gebunden ist.

3. Zusammensetzung nach einem der vorangehenden Ansprüche, **dadurch gekennzeichnet, dass** der Zielzell-spezifische Bestandteil ein Antikörper oder ein Antikörper-Fragment oder ein Wachstumsfaktor, Zytokin oder Transferrin oder eine Kombination von irgendeinem der vorangehenden ist.

4. Zusammensetzung nach einem der vorangehenden Ansprüche, wobei das Saponin Saponinum album von Gypsophila paniculata ist.

5. Zusammensetzung nach einem der vorangehenden Ansprüche, **dadurch gekennzeichnet, dass** die Zusammensetzung weiterhin wenigstens eine der folgenden funktionellen Einheiten umfasst: ein Transportprotein, das in der Lage ist, das pharmakologisch aktive Mittel in eine Zelle zu transportieren, eine zytosolische spaltbare Bindung, eine endosomale spaltbare Bindung.

6. Zusammensetzung nach Anspruch 5, **dadurch gekennzeichnet, dass** das pharmakologisch aktive Mittel an den Zielzell-spezifischen Bestandteil über das Transportprotein, die zytosolische spaltbare Bindung und/oder die endosomale spaltbare Bindung gekoppelt ist.

7. Zusammensetzung nach einem der Ansprüche 5-6, **dadurch gekennzeichnet, dass** das Transportprotein eine Protein-Transduktions-Domäne (PTD) ist, die zytosolische spaltbare Bindung Teil eines Peptids ist und die endosomale spaltbare Bindung Teil eines Peptids ist.

8. Zusammensetzung nach einem der vorangehenden Ansprüche zur Verwendung als ein Medikament.

9. Verwendung einer Zusammensetzung nach einem der vorangehenden Ansprüche zur Herstellung eines Medikaments zur Behandlung eines Tieres mit einer Krankheit, ausgewählt aus der Gruppe, umfassend Krebserkrankungen.

10. Verwendung nach Anspruch 9, **dadurch gekennzeichnet, dass** das Saponin separat von dem pharmakologisch aktiven Mittel, das an den Zielzell-spezifischen Bestandteil gekoppelt ist, verabreicht werden soll.

11. Verwendung nach einem der Ansprüche 9-10, **dadurch gekennzeichnet, dass** das Saponin über eine Route verabreicht werden soll, die unterschiedlich zu der Verabreichungsroute des pharmakologisch aktiven Mittels ist, das an den Zielzell-spezifischen Bestandteil gekoppelt ist.

12. Verwendung nach Anspruch 11, **dadurch gekennzeichnet, dass** das Saponin über eine Injektion verabreicht werden soll und das pharmakologisch aktive Mittel, gekoppelt an den Zielzell-spezifischen Bestandteil, über Einnahme verabreicht wird, oder umgekehrt.

13. Verwendung nach einem der Ansprüche 9-10, **dadurch gekennzeichnet, dass** das Saponin auf die selbe Route wie das pharmakologisch aktive Mittel, gekoppelt an den Zielzell-spezifischen Bestandteil, verabreicht werden soll.

14. Verwendung nach Anspruch 13, **dadurch gekennzeichnet, dass** die Verabreichung mittels Injektion erfolgt.

15. Verwendung nach einem der Ansprüche 9-14, **dadurch gekennzeichnet, dass** das Saponin vor dem pharmakologisch aktiven Mittel, gekoppelt an den Zielzell-spezifischen Bestandteil, verabreicht werden soll.

16. Kit, umfassend in einem oder mehreren Behältern die Zusammensetzung nach einem der Ansprüche 1-8.

17. Kit nach Anspruch 16, **dadurch gekennzeichnet, dass** das pharmakologisch aktive Mittel, gekoppelt an den Zielzell-spezifischen Bestandteil, und das Saponin in separaten Behältern sind.

## Revendications

1. Composition comprenant
au moins un agent pharmacologiquement actif qui présente un effet pharmacologique détectable sur une cellule ou un organisme, couplé à un composant spécifique d'une cellule cible qui se lie spécifiquement à une cellule cible, et
au moins une saponine, ladite saponine étant choisie dans le groupe comprenant des saponines triterpénoïques avec des structures basiques appartenant au type 12,13-déshydro-oléanane avec une fonction aldéhyde en position 23,
dans laquelle ledit agent pharmacologiquement actif couplé au dit composant spécifique de la cellule cible est une toxine chimérique ou un conjugué de toxine, ladite toxine chimérique ou ledit conjugué de toxine étant une protéine recombinante simple,
dans laquelle ledit agent pharmacologiquement actif n'est pas une saponine, et n'est pas une protéine de liaison d'antigène comprenant une région de liaison d'antigène et une région ou des régions d'un anticorps qui interviennent dans des processus immunologiques dépendant de l'anticorps, comme la cytotoxicité cellulaire dépendante de l'anticorps, l'activation du complément, l'opsonisation et la phagocytose, dans laquelle ledit composant spécifique de la cellule cible est choisi dans le groupe comprenant (a) des protéines, comme des protéines naturelles, des protéines recombinantes, des anticorps, des fragments d'anticorps, des lipoprotéines et des ligands de protéines, comme des facteurs de croissance, par exemple l'EGF, le GM-CSF, le VEGF et le TGF et des chimères de ceux-ci, des cytokines, par exemple des interleukines, des interférons, la transférine, des molécules d'adhérence, par exemple le CD4 et l'activateur du plasminogène de type urokinase, (b) des peptides, comme des peptides naturels et des peptides synthétiques, (c) des hormones et des combinaisons de (a) à (c), et dans laquelle ladite cellule cible est choisie dans le groupe comprenant des cellules cancéreuses, et dans laquelle ledit agent pharmacologiquement actif est une cytotoxine qui est choisie dans le groupe comprenant (a) des protéines végétales toxiques, comme des protéines d'inactivation des ribosomes (RIP), en particulier des protéines de type I, par exemple la saporine, la dianthine, la gélonine, la PAP et des fragments actifs sur le plan enzymatique de celles-ci et la chaîne A de protéines de type II, par exemple la ricine, l'abrine, la lectine du gui (viscumine), la modeccine mais en n'excluant pas d'autres fragments de protéines de type II ou des protéines de type II entières, (b) des protéines et des peptides fongiques toxiques, comme l'a-sarcine ou la mitogilline, (c) des toxines bactériennes, comme la toxine diphtérique ou l'exotoxine de *Pseudomonas,* et (d) des protéines animales toxiques, comme l'angiogénine ou le granzyme B, en particulier d'origine humaine.

2. Composition selon la revendication 1, **caractérisée en ce que**, dans ladite toxine chimérique ou ledit conjugué de toxine, ledit agent pharmacologiquement actif est couplé au dit composant spécifique de la cellule cible par l'intermédiaire d'une liaison covalente.

3. Composition selon l'une quelconque des revendications précédentes, **caractérisée en ce que** ledit composant spécifique de la cellule cible est un anticorps ou un fragment d'anticorps ou un facteur de croissance, une cytokine ou la transférine ou une combinaison de n'importe lesquels de ceux-ci.

4. Composition selon l'une quelconque des revendications précédentes, dans laquelle ladite saponine est la saponine blanche de *Gypsophila paniculata.*

5. Composition selon l'une quelconque des revendications précédentes, **caractérisée en ce que** ladite composition comprend en outre au moins l'une des unités fonctionnelles suivantes : une protéine de transport capable de transporter ledit agent pharmacologiquement actif dans une cellule, une liaison cytosolique clivable, une liaison endosomiale clivable.

6. Composition selon la revendication 5, **caractérisée en ce que** ledit agent pharmacologiquement actif est couplé au dit composant spécifique de la cellule cible par l'intermédiaire de ladite protéine de transport, de ladite liaison cytosolique clivable et/ou de ladite liaison endosomiale clivable.

7. Composition selon l'une quelconque des revendications 5 ou 6, **caractérisée en ce que** ladite protéine de transport est un domaine de transduction de protéine (PTD), ladite liaison cytosolique clivable fait partie d'un peptide et ladite liaison endosomiale clivable fait partie d'un peptide.

8. Composition selon l'une quelconque des revendications précédentes, pour une utilisation en tant que médicament.

9. Utilisation d'une composition selon l'une quelconque des revendications précédentes pour la fabrication d'un médicament destiné au traitement d'un animal souffrant d'une maladie choisie dans le groupe comprenant des maladies cancéreuses.

10. Utilisation selon la revendication 9, **caractérisée en ce que** ladite saponine doit être administrée séparément dudit agent pharmacologiquement actif couplé au dit composant spécifique de la cellule cible.

11. Utilisation selon l'une quelconque des revendications 9 ou 10, **caractérisée en ce que** ladite saponine doit être administrée par une voie différente de la voie d'administration dudit agent pharmacologiquement actif couplé au dit composant spécifique de la cellule cible.

12. Utilisation selon la revendication 11, **caractérisée en ce que** ladite saponine doit être administrée par injection et ledit agent pharmacologiquement actif couplé au dit composant spécifique de la cellule cible est administré par ingestion ou vice versa.

13. Utilisation selon l'une quelconque des revendications 9 à 10, **caractérisée en ce que** la saponine doit être administrée par la même voie que ledit agent pharmacologiquement actif couplé au dit composant spécifique de la cellule cible.

14. Utilisation selon la revendication 13, **caractérisée en ce que** ladite administration se produit par injection.

15. Utilisation selon l'une quelconque des revendications 9 à 15, **caractérisée en ce que** ladite saponine doit être administrée avant ledit agent pharmacologiquement actif couplé au dit composant spécifique de la cellule cible.

16. Kit comprenant, dans un ou plusieurs récipients, ladite composition selon l'une quelconque des revendications 1 à 8.

17. Kit selon la revendication 16, **caractérisé en ce que** ledit agent pharmacologiquement actif couplé au dit composant spécifique de la cellule cible et ladite saponine sont dans des récipients séparés.
